# EUROPEAN PATENT SPECIFICATION

(11) **EP 1 536 727 B1**
(45) Date of publication and mention of the grant of the patent: **04.12.2013**
(21) Application number: 03793358.7
(22) Date of filing: 25.08.2003
(51) Int. Cl.: A61B 8/12, A61B 5/0456, A61B 8/08, A61B 8/00, A61B 5/02, G06T 7/00

(54) **SYSTEM AND METHOD OF ACQUIRING BLOOD-VESSEL DATA**
SYSTEM UND VERFAHREN ZUR ERFASSUNG VON BLUTGEFÄSSDATEN
SYSTEME ET PROCEDE POUR L'ACQUISITION DE DONNEES SUR UN VAISSEAU SANGUIN

(30) Priority: 26.08.2002 US 406183 P; 26.08.2002 US 406254 P; 26.08.2002 US 406184 P; 26.08.2002 US 406185 P; 26.08.2002 US 406234 P
(43) Date of publication of application: 08.06.2005
(73) Proprietor: THE CLEVELAND CLINIC FOUNDATION, Cleveland, OH 44195 (US)
(72) Inventor: KUBAN, Barry, D., Avon Lake, OH 44012 (US); KLINGENSMITH, Jon, D., Shaker Heights, OH 44120 (US); VINCE, D., Geoffrey, Avon Lake, OH 44012 (US); NAIR, Anuja, Cleveland Heights, OH 44106 (US)
(74) Representative: Jansen, Cornelis Marinus
(86) International application number: PCT/US2003/026492
(87) International publication number: WO 2004/017821

(56) References cited:
- WO-A-01/20552
- WO-A-02/064011
- US-A- 4 917 097
- US-A- 5 588 432
- US-A- 5 771 895
- US-A- 6 148 095
- US-A- 6 152 878

## Description

### BACKGROUND OF THE INVENTION

### 1. Field of the Invention

The present invention relates to acquiring blood-vessel data, or more particularly, to a system and method of substantially synchronizing the acquisition of blood-vessel data to an identifiable portion of heartbeat data.

### 2. Description of Related Art

Blood-vessel data (e.g., data that can be used to identify the shape of a blood vessel, its density, its composition, etc.) can provide useful information in the diagnoses and/or treatment of a patient. For example, intra-vascular ultrasound (IVUS) devices use blood-vessel data to reproduce or image a blood vessel. Specifically, a transducer is attached to a distal end of a catheter and electrically connected to an IVUS device. The transducer is then placed inside a particular blood vessel and used to transmit acoustic signals. The reflections of these signals are then received by the transducer, converted into electrical signals, and transmitted to the IVUS device. The electrical signals are then used to create an image of the blood vessel (or a portion thereof).

Blood vessels, however, are continuously expanding and relaxing in response to blood being pumped there through. Thus, by continuously gathering blood-vessel data, a blood vessel, as it expands and relaxes, can be imaged. If, however, the blood vessel needs to be monitored in a particular position (e.g., to image the blood vessel as if it were standing still--i. e., not expanding and relaxing), it may be necessary to acquire the blood-vessel data when the blood vessel's shape is substantially uniform (i. e. , when the blood vessel is in a particular position).
The traditional method of doing this (at least with respect to an IVUS device) is to gather both blood-vessel and heartbeat data (e. g., EKG data), use the blood-vessel data to generate real-time images (i. e., video of the expanding and contracting blood vessel), record these images onto a VHS tape, and use a computer and the heartbeat data to extract relevant frames from the VHS tape. The heartbeat data is used by the computer because the rhythm of the heart is related to the expansion and contraction of the blood vessels. Thus, by extracting the frames recorded during an identifiable period in the heart's cycle, the blood vessel can be monitored as if it were standing still--i. e., not expanding and contracting.

The drawbacks of this method is that image resolution is lost when the data is recorded onto the VHS tape. Furthermore, this method is extremely time consuming. Not only is unnecessary data (i. e., data unrelated to the identifiable period) gathered and recorded onto the VHS tape, but processing time is necessary to extract the relevant frames from the VHS tape. Thus, a need exists for a system and method of acquiring blood-vessel data from a blood vessel in a particular position that overcomes at least one of these drawbacks.

An embodiment of a medical imaging device is known from WO 02/064011. In the known device a processor coupled with a display unit and a database is provided. The known medical imaging device further comprises a medical positioning system coupled to said processor arranged for tracing a surgical tool. The known device further comprises a two-dimensional imaging system coupled to said processor and a an inspected org monitor interface coupled to said processor and to an organ monitor for providing an origan timing signal associated with an inspected organ. The known device further comprises a superimposing processor coupled to said processor.

In operation, the superimposing computer receives each of the two-dimensional images, the respective three-dimensional location and orientation of that specific two-dimensional image and the organ timing signal of the organ (heart) at the time the image was captured. The superimposing computer associates each detected two-dimensional image with the location and orientation information of the organ-timing signal.

An embodiment of a catheter suitable for imaging is known from US 5, 588, 432. Using the known catheter ultrasonic imaging frames that go through one complete cardiac cycle from systole to diastole and back to systole are superimposed on the ultrasound imaging frames at any given location.

A further embodiment of an ultrasonic blood flow sensing apparatus is known from US 4, 501, 279. In the known apparatus B scan tomogram is produced wherein an ECG gate signal is used as a sampling signal for read and write operations to a suitable random access memory (RAM).

An embodiment of a method for rendering medical images in real time is known from WO 01/20552. In the known method an MRI-based system enabling reconstruction of a 3D heart model is used for investigating a structure of the human heart including walls and chambers.

In the known method an ECG grated MRI acquisition is used, wherein an ECG gating signal is used for triggering the MRI scanner at the peak of the R wave in the QRS complex.

An embodiment of a catheter for obtaining three-dimensional reconstruction of a vascular lumen and wall is known from US 5, 771, 895. The known catheter is an IVUS system which cooperates with a heart-monitoring device arranged to acquire heartbeat data for enabling a three-dimensional reconstruction of a body vessel during post-processing. For that purpose end diastolic images from the IVUS device are post-processed.

### SUMMARY OF THE INVENTION

The present invention provides a system for substantially synchronizing the acquisition of blood-vessel data to an identifiable portion of heartbeat data as is recited in claim 1. Preferred embodiments of the present invention operate in accordance with a heart-monitoring device, a data-gathering device, and a data- gathering probe. Specifically, a data-gathering device is adapted to acquire heartbeat data and blood-vessel data from a heart-monitoring device and a data- gathering probe, respectively. In the present invention, the blood-vessel data is acquired during a cyclical portion of the heartbeat data. As previously discussed, it is the contraction and relaxation of the heart muscles (or the blood that flows as a result thereof) that causes the blood vessels to expand and relax. Thus, it is possible to identify a particular position (or shape) of a blood vessel by identifying a corresponding portion of the heart's repetitive cycle. This information (i.e., the identified portion of the heartbeat data) can be used to acquire blood-vessel data (or multiple sets thereof) from a blood vessel having a substantially uniform shape. In other words, by identifying a cyclical (or commonly reoccurring) portion of heartbeat data and acquiring blood-vessel data during this cyclical portion (or during an interval or time period that substantially corresponds thereto), the blood vessel can be analyzed as if it were standing still -- i.e., not expanding and relaxing.

In one embodiment of the present invention, the heart-monitoring device includes an EKG device. An EGK device uses a plurality of electrodes to measure electrical current passing through a patient's body. The electrical current corresponds to the electrical activity of the patient's heart muscles, or the contraction and relaxation thereof. This current (or related data) can be used to identify a cyclical portion of the heart's cycle, thus allowing blood-vessel data to be acquired during intervals that substantially correspond thereto (i.e., when the blood vessel is in a substantially uniform position).

In another embodiment of the present invention, the data-gathering device includes an intra-vascular ultrasound (IVUS) device and a computing device. In this embodiment, the IVUS device is adapted to receive blood-vessel data from the data-gathering probe (either continuously or during intervals that substantially correspond to cyclical periods of heartbeat data). The blood-vessel data (or data resulting therefrom) is then acquired by the computing device (e.g., received and/or stored) during intervals that substantially correspond to cyclical periods of heartbeat data.

In another embodiment of the present invention, the data-gathering device includes an IVUS device or a computing device. In this embodiment, blood-vessel data is received and/or stored by the data-gathering device during intervals that substantially correspond to cyclical portions of heartbeat data.

In another embodiment of the present invention, the data-gathering probe includes at least one transducer attached to a distal end of a catheter, where the catheter further includes a data-transmission circuit for transmitting (and receiving) electrical signals to (or from) the transducer(s). In this embodiment, the transducer is placed inside a blood vessel and used to gather blood-vessel data by transmitting and receiving acoustic waves.

The data-gathering system further includes a retraction device. Specifically, the retraction device is attached to the data-gathering probe and used to move the probe though a blood vessel. In one embodiment of the present invention, the retraction device is further adapted to move the probe through the blood vessel at a substantially steady speed.

A more complete understanding of the system and method of substantially synchronizing the acquisition of blood-vessel data to an identifiable portion of heartbeat data will be afforded to those skilled in the art, as well as a realization of additional advantages and objects thereof, by a consideration of the following detailed description of the preferred embodiment. Reference will be made to the appended sheets of drawings which will first be described briefly.

### BRIEF DESCRIPTION OF THE DRAWINGS

Figure 1 illustrates a data-gathering system that substantially synchronizes the acquisition of blood-vessel data to an identifiable portion of heartbeat data.
Figure 1A illustrates an interval (T1) that substantially corresponds to an exemplary cyclical portion of heartbeat data (e.g., EKG data).
Figure 2 further illustrates the data-gathering device depicted in Figure 1.
Figure 3 illustrates a data-gathering probe including a plurality of transducer and located within a blood vessel.
Figure 4 illustrates a catheter having a data-transmission circuit and a transducer attached thereto, said transducer being adapted for rotation.
Figure 5 illustrates a catheter having a data-transmission circuit and a plurality of transducers attached thereto.

### DETAILED DESCRIPTION OF THE PREFERRED EMBODIMENT

The present invention provides a system for substantially synchronizing the acquisition of blood-vessel data to an identifiable portion of heartbeat data. In the detailed description that follows, like element numerals are used to describe like elements illustrated in one or more figures.

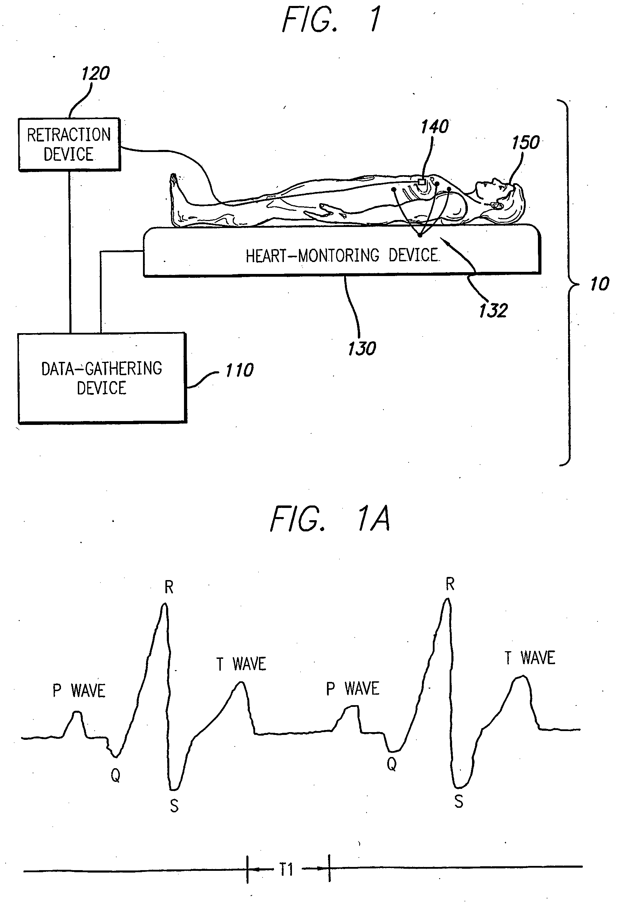

Preferred embodiments of the present invention operate in accordance with a heart-monitoring device, a data-gathering device, and a data-gathering probe. Figure 1 illustrates a data-gathering system 10 in accordance with one embodiment of the present invention. In this embodiment, a data-gathering device 110 is electrically connected to a heart-monitoring device 130, which is attached to a patient 150 via at least one heart-monitoring probe 132. The heart-monitoring device 130 is used to gather heartbeat data (e.g., data related to the contraction and/or relaxation of the heart muscles, data related to the volume and/or pressure of blood flowing as a result thereof, etc.) from the patient 150. This heartbeat data is then provided to (or acquired by) the data-gathering device 110. It should be appreciated that the data-gathering device depicted in Figure 1 includes, but is not limited to, ultrasonic devices (e.g., an intra-vascular ultrasound (IVUS) console), thermographic devices, optical devices (e.g., an optical coherence tomography (OCT) console), MRI devices, computing devices (e.g., a personal computer, a general purpose computing device, an application specific computing device, etc.), and/or any other data gathering devices (including combinations thereof) that are generally known to those skilled in the art. It should further be appreciated that the heart-monitoring device depicted in Figure 1 includes, but is not limited to, electrocardiograph (EKG) devices, pressure-monitoring devices, or any other heart monitoring device that is generally known to those skilled in the art and can be used to monitor the heart's cycle (or data related thereto -- e.g., pressure levels, electrical signals, etc.).

The data-gathering device 110 is further electrically connected to a data-gathering probe 140, which is inserted into a blood vessel (not shown) of the patient 150. The data-gathering probe 140 is used to gather blood-vessel data (e.g., data that can be used to identify the shape of a blood vessel, its density, its composition, etc.). This data (or data related thereto) is then provided to (or acquired by) the data-gathering device 110. It should be appreciated that the data-gathering probe includes, but is not limited to, at least one transducer or any other reception device that is generally known to those skilled in the art. Thus, for example, the use of any reception device adapted to acquire data (e.g., reflected data, etc.), regardless of whether the data is thermal, optical, acoustical, electrical, etc., is within the scope of the present invention. It should further be appreciated that the number and/or location of components depicted In Figure 1 are not intended to limit the present invention, but are merely provided to illustrate the environment in which the present invention operates. Thus, for example, a data-gathering system including multiple data-gathering devices, multiple data-gathering probes, and/or additional or fewer components is within the scope of the present invention.

In the present Invention, the blood-vessel data is acquired during a cyclical portion of the heartbeat data. As previously discussed, it is the contraction and relaxation of the heart muscles (or the blood that flows as a result thereof) that causes the blood vessels to expand and relax. Thus, it is possible to identify a particular position (or shape) of a blood vessel by identifying a corresponding portion of the heart's repetitive cycle. This information (i.e., the identified portion of the heartbeat data) can be used to acquire blood-vessel data (or multiple sets thereof) from a blood vessel having a substantially uniform shape. In other words, by identifying a cyclical (or commonly reoccurring) portion of heartbeat data and acquiring blood-vessel data during this cyclical portion (or during an Interval or time period that substantially corresponds thereto), the blood vessel can be analyzed as if it were standing still - I.e., not expanding and relaxing. It should be appreciated that the term "acquire" (or any variation thereof), as that term is used herein, should be construed broadly to include the reception and/or storage of data. Thus, for example, a data-gathering device (or a portion thereof) adapted to receive and/or store blood-vessel data (or data related thereto) during a cyclical portion of the heartbeat data is within the scope of the present invention.

In one embodiment of the present invention, the heart-monitoring device includes an EKG device. An EKG device uses a plurality of electrodes to measure electrical current passing through a patient's body. The electrical current corresponds to the electrical activity of the patient's heart muscles, or the contraction and relaxation thereof. By plotting or imaging this current, the heart's rhythm (or cycles) can be observed. An example of such an image (i.e., multiple sets of heartbeat data) is illustrated in Figure 1A. Specifically, each heart cycle includes a P wave, a T wave, and points Q, R and S. These identifiable portions make it possible to identify a cyclical (or commonly reoccurring) portion of the heart's cycle (or heartbeat data).

For example, the portion between the end of the T wave and the beginning of the P wave can be identified as a cyclical portion of heartbeat data having a corresponding interval T1. This portion, or more particular its interval T1, can be used to acquire blood-vessel data from a uniformly shaped blood vessel. This is because the interval T1 (which reoccurs periodically) substantially corresponds to the identified cyclical portion, which substantially corresponds to a blood vessel having a particular shape or position. It should be appreciated that, while it may be advantageous to identify certain cyclical portions of heartbeat data, the present invention is not limited to the identification of any particular cyclical portion. It should further be appreciated that the term "portion" (or any variation thereof), as that term is used herein, should be construed broadly to include both segments and points of heartbeat data. Furthermore, it should also be appreciated that the terms "interval" and "time period" (or any variations thereof), as these terms are used herein, should be construed broadly to include both passages of time and points in time. Thus, for example, identifying the point "Q" as a cyclical portion of heartbeat data, which has a corresponding interval, or a corresponding point in time (as opposed to a passage of time), is within the scope of the present invention.

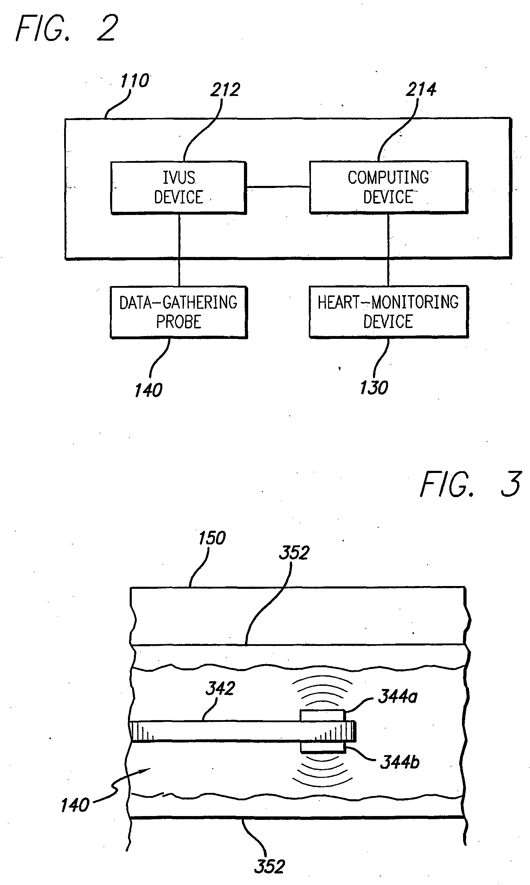

In one embodiment of the present invention, the data-gathering device includes both an IVUS device and a computing device. Specifically, as shown in Figure 2, the data-gathering device 110 includes an IVUS device 212 electrically connected to the data-gathering probe 140 and a computing device 214 electrically connected to the heart-monitoring device 130. Thus, the computing device 214 is adapted to acquire (via the IVUS device 212) blood-vessel data (or data related thereto) during intervals that correspond to cyclical portions of heartbeat data. It should be appreciated that the phrase "blood-vessel data," as that phrase is used herein, is to be construed broadly and includes the blood-vessel data gathered by the data-gathering probe and any blood- vessel data related thereto or created therefrom (e.g., as processed by the IVUS device). It should further be appreciated that, in this embodiment, it is the computing device 214 that is adapted to acquire blood-vessel data during intervals that correspond to cyclical portions of heartbeat data. Thus, while the IVUS device may also be adapted to acquire blood-vessel data during these intervals, an IVUS device adapted to continuously receive heartbeat data is within the scope of the present invention.

In one embodiment of the present invention, the data-gathering probe includes at least one transducer. Specifically, as shown in Figure 3, a plurality of transducers 344a, 344b, are attached to a distal end of a catheter 342 having a data-transmission circuit located therein (not shown). In this embodiment, the transducers 344a, 344b are placed inside a blood vessel 352 of the patient 150 and used to gather blood-vessel data. Specifically, each transducer is adapted to (i) convert electrical signals into acoustic waves, (ii) transmit the acoustic waves, (iii) receive any reflections thereof, and (iv) convert the reflections into electrical signals. In this embodiment, the electrical signals are propagated over (i.e., received from and transmitted over) the data-transmission circuit (not shown).

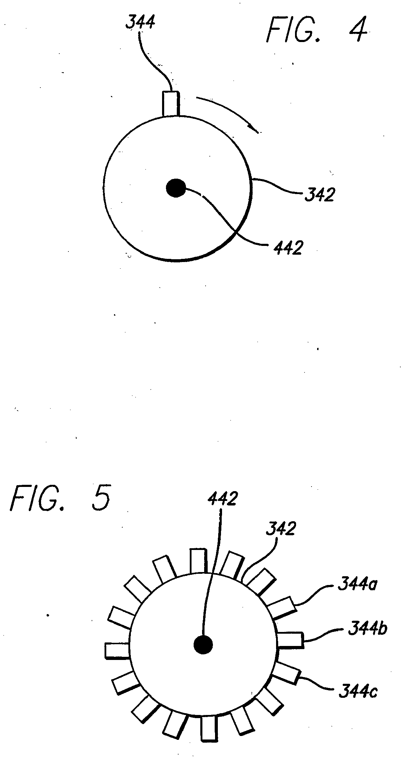

In another embodiment of the present the transducer is further adapted to rotate. Specifically, as shown in Figure 4, a transducer 344 is attached to the distal end of a catheter 342 having a data-transmission circuit 442. In this embodiment of the present invention, the transducer 344 is adapted to rotate around the catheter 342 and receive blood-vessel data from a variety of angular positions, or rotational orientations. In one embodiment of the present invention, the transducer is adapted to start at a particular rotational orientation and rotate (and acquire blood-vessel data) in response (either directly or indirectly) to the transmission of probe-triggering data (e.g., by the data-gathering device). Such an embodiment allows the data-gathering device to be synchronized with the rotational orientation of the transducer.

In another embodiment of the present invention, the transducer is adapted to continuously rotate and continuously gather blood-vessel data. In this embodiment, the data-gathering device may be adapted to identify at least one rotational orientation of the transducer (e.g., a starting rotational orientation). This is because the data-gathering device needs to understand the rotational orientation of the blood-vessel data being acquired. One method of doing this is to start acquiring data when the transducer is in a known rotational orientation (e.g., a starting rotational orientation). Thus, for example, if the transducer is adapted to rotate to two-hundred and fifty-six positions per cycle, and the transducer is continuously rotating and acquiring data, then the data-gathering device may be adapted to identify when the transducer is rotationally oriented in its "starting position," and gather the next two-hundred and fifty-six items of blood-vessel data.

In another embodiment of the present invention, a plurality of transducers are located around a catheter. Specfically, as shown in Figure 5, a plurality of transducers (e.g., 344a, 344b, 344c, etc.) are circumferentially spaced around the distal end of a catheter 342. This allows multiple items of blood-vessel data to be transmitted via the data-transmission circuit 442 (either serially or in parallel). It further eliminates the need for each transducer to rotate. It should be appreciated that the number of transducers depicted in Figure 5 is not intended to limit the present invention, but is merely provided to identify the environment in which the present invention may operate. Thus, a data-gathering probe having more or less transducers is within the scope of the present invention.

The data-gathering system further includes a retraction device. Specifically, as shown in Figure 1, a retraction device 120 is attached to the data-gathering device 110 and adapted to move the data-gathering probe 140 though the blood vessel. It should be appreciated that the data-gathering probe 140, which is physically connected to the retraction device, may be electrically connected to the data-gathering device 110 either directly (not shown) or indirectly (e.g., via the retraction device 120).

In one embodiment of the present invention, the retraction device 120 is further adapted to move the data-gathering probe 140 through the blood vessel at a substantially stead speed. This allows, for example, the data-gathering device 110 to image a blood vessel section (either In two-dimensional or three-dimensional form). Specifically, by acquiring blood-vessel data during intervals that correspond to cyclical portions of heartbeat data and knowing the linear rate at which this data is being acquired (e.g., by providing the speed to, or receiving it from, the data-gathering device), the blood vessel can effectively be recreated or imaged.

It should be appreciated that blood-vessel data can be used in a number of application including, but not limited to, diagnosing and/or treating patients. For example, blood-vessel data can be used to identify and/or image blood vessel boarders or boundaries, as provided by U.S. Provisional Application Numbers, 60/406,184, 60/406,234 and 60/406,185, all of which were filed August 26, 2002, and by U.S. Patent Number 6,381,350, which issued April 30, 2002. Another use for blood-vessel data is for classifying and/or imaging vascular plaque, as provided by U.S. Provisional Application Numbers, 60/406,254 and 60/406,148, filed August 26, 2002, and by U.S. Patent Number 6,200,268, which issued March 13, 2001.

Having thus described a preferred embodiment of a system and method of substantially synchronizing the acquisition of blood-vessel data to an identifiable portion of heartbeat data, it should be apparent to those skilled in the art that certain advantages of the system have been achieved. It should also be appreciated that various modifications, adaptations, and alternative embodiments thereof may be made within the scope of the present invention. For example, a computing device could be adapted to receive blood-vessel data directly from a data-gathering probe (as oppose to receiving it via an IVUS device). The invention is further defined by the following claims.

## Claims

1. A system for acquiring blood-vessel data (10), comprising:
a data-gathering probe (140) adapted to acquire blood-vessel data;
a heart-monitoring device (130) adapted to acquire heartbeat data; and
a data-gathering device (110) connected to said data-gathering probe (140) and said heart-monitoring device (130), the data-gathering device adapted to:
acquire said heartbeat data from the heart-monitoring device;
identify a cyclical portion (T1) of said heartbeat data, said cyclical portion being substantially common to multiple sets of heartbeat data; and
acquire said blood-vessel data from the data-gathering probe (140) during an interval substantially corresponding to said cyclical portion (T1) of said heartbeat data; wherein the data-gathering device (110) acquires said blood-vessel data during the interval in response to a probe-trigger provided once per heart beat marking a beginning of the cyclical portion occurring between identifiable portions of each heart cycle;
the system being **characterized by**:
the data-gathering probe (140) being adapted to be positioned within a blood vessel and to acquire blood-vessel data from within the blood vessel;
a retraction device (120) adapted to move said data-gathering probe (140) through the blood vessel at a substantially constant speed, wherein the data-gathering probe (140) is arranged to gather data while being moved at the substantially constant speed;
the data-gathering device (110) acquiring said blood-vessel data only during the interval while the data-gathering probe is moved through the blood vessel at the substantially constant speed in response to a probe-trigger provided once per heart beat marking a beginning of the cyclical portion occurring between identifiable portions of each heart cycle

2. A system according to Claim 1, wherein the blood vessel data comprises blood-vessel data suitable to identify a shape of a blood vessel, a density of the blood vessel, or a composition of the blood vessel.

3. A system according to Claim 1 or 2, wherein said interval corresponds to a condition of a uniformly shaped blood vessel.

4. The system of Claim 1 or 2, further comprising a catheter (342), said data-gathering probe being attached to a distal end of said catheter.

5. The system of Claim 4, wherein said data-gathering probe (132) comprises a plurality of transducers (344a, 344b, 344c, etc.) spaced circumferentially around said distal end of said catheter (342) and adapted to receive at least said blood-vessel data.

6. The system of Claim 4, wherein said data-gathering probe (132) further comprises at least one transducer adapted to rotate and receive at least said blood-vessel data.

7. The system of Claim 1, 2 or 3, wherein said heart-monitoring device comprises an electrocardiograph (EKG) device.

8. The system of Claim 1, 2 or 3, wherein said data-gathering device comprises a programmable computing device.

9. The system of Claim 1, 2 or 3, wherein said data-gathering device comprises an intra-vascular ultrasound (IVUS) device.

10. The system of Claim 8, wherein said data-gathering device further comprises an intra-vascular ultrasound (IVUS) device.

11. The system of Claim 6, wherein said data-gathering device is further adapted to start acquiring said blood-vessel data when said at least one transducer is rotationally oriented in a predetermined position.

## Patentansprüche

1. System zur Erfassung von Blutgefäßdaten (10), umfassend eine Datensammelsonde (140), geeignet zum Erfassen von Blutgefäßdaten; eine Herzüberwachungsvorrichtung (130), geeignet zum Erfassen von Herzschlagdaten;
und
eine Datensammelvorrichtung (110), verbunden mit der Datensammelsonde (140) und der Herzüberwachungsvorrichtung (130), wobei die Datensammelvorrichtung geeignet ist zum:
Erfassen von Herzschlagdaten von der Herzüberwachungsvorrichtung;
Identifizieren eines zyklischen Teils (T1) der Herzschlagdaten, wobei der zyklische Teil im Wesentlichen mehreren Sätzen an Herzschlagdaten gemeinsam ist;
und
Erfassen der Blutgefäßdaten von der Datensammelsonde (140) während eines Intervalls, der im Wesentlichen dem zyklischen Abschnitt (T1) der Herzschlagdaten entspricht; wobei die Datensammelvorrichtung (110) die Blutgefäßdaten während des Intervalls in Reaktion auf einen Sondenauslöser, bereitgestellt einmal pro Herzschlag, markierend den Beginn des zyklischen Abschnitts, der zwischen identifizierbaren Abschnitten jedes Herzzyklus erfolgt, erfasst;
welches System **dadurch gekennzeichnet ist, dass**:
die Datensammelsonde (140) geeignet ist, um in einem Blutgefäß positioniert zu werden und Blutgefäßdaten aus dem Blutgefäß zu erfassen;
eine Rückzugsvorrichtung (120), geeignet zum Bewegen der Datensammelsonde (140) durch das Blutgefäß mit einer im Wesentlichen konstanten Geschwindigkeit, wobei die Datensammelsonde (140) geeignet ist, um während sie mit der im Wesentlichen konstanten Geschwindigkeit bewegt wird, Daten zu sammeln;
die Datensammelvorrichtung (110) die Blutgefäßdaten nur während des Intervalls erfasst, in dem die Datensammelsonde in Reaktion auf einen Sondenauslöser, bereitgestellt einmal pro Herzschlag, markierend einen Beginn des zyklischen Abschnitts, der zwischen identifizierbaren Abschnitten jedes Herzzyklus erfolgt, mit der im Wesentlichen konstanten Geschwindigkeit bewegt wird.

2. System nach Anspruch 1, wobei die Blutgefäßdaten geeignete Blutgefäßdaten zum Identifizieren einer Form eines Blutgefäßes, einer Dichte des Blutgefäßes oder einer Zusammensetzung des Blutgefäßes umfassen.

3. System nach Anspruch 1 oder 2, wobei das Intervall einem Zustand eines einheitlich geformten Blutgefäßes entspricht.

4. System nach Anspruch 1 oder 2, ferner umfassend einen Katheter (342), wobei die Datensammelsonde an einem distalen Ende des Katheters angebracht ist.

5. System nach Anspruch 4, wobei die Datensammelsonde (132) mehrere Messfühler (344a, 344b, 344c usw.) umfasst, die ringsum das distale Ende des Katheters (342) angeordnet sind und geeignet sind, um mindestens die Blutgefäßdaten zu empfangen.

6. System nach Anspruch 4, wobei die Datensammelsonde (132) ferner mindestens einen Messfühler umfasst, der zum Drehen und zum Empfangen von mindestens den Blutgefäßdaten geeignet ist.

7. System nach Anspruch 1, 2 oder 3, wobei die Herzüberwachungsvorrichtung eine Elektrokardiograph (EKG)-Vorrichtung umfasst.

8. System nach Anspruch 1, 2 oder 3, wobei die Datensammelvorrichtung eine programmierbare Rechenvorrichtung umfasst.

9. System nach Anspruch 1, 2 oder 3, wobei die Datensammelvorrichtung eine intravaskuläre Ultraschall (IVUS)-Vorrichtung umfasst.

10. System nach Anspruch 8, wobei die Datensammelvorrichtung ferner eine intravaskuläre Ultraschall (IVUS)-Vorrichtung umfasst.

11. System nach Anspruch 6, wobei die Datensammelvorrichtung ferner geeignet ist, die Erfassung der Blutgefäßdaten zu beginnen, wenn mindestens ein Messfühler in einer vorbestimmten Position ausgerichtet ist.

## Revendications

1. Système pour acquérir des données de vaisseau sanguin (10), comprenant :
une sonde de recueil de données (140) adaptée pour acquérir des données de vaisseau sanguin ;
un dispositif de surveillance cardiaque (130) adapté pour acquérir des données de fréquence cardiaque ; et
un dispositif de recueil de données (110) relié à la sonde de recueil de données (140) et au dispositif de surveillance cardiaque (130), le dispositif de recueil de données étant adapté pour :
acquérir les données de fréquence cardiaque du dispositif de surveillance cardiaque ;
identifier une partie cyclique (T1) des données de fréquence cardiaque, la partie cyclique étant sensiblement commune à de multiples ensembles de données de fréquence cardiaque ; et
acquérir les données de vaisseau sanguin à partir de la sonde de recueil de données (140) pendant un intervalle qui correspond sensiblement à la partie cyclique (T1) des données de fréquence cardiaque , dans lequel le dispositif de recueil de données (110) acquiert les données de vaisseau sanguin au cours de l'intervalle en réponse à un déclenchement de sonde fourni une fois par battement cardiaque marquant un début de la partie cyclique se produisant entre des parties identifiables de chaque cycle cardiaque ;
le système étant **caractérisé en ce que** :
la sonde de recueil de données (140) est adaptée pour être positionnée à l'intérieur d'un vaisseau sanguin et pour acquérir des données de vaisseau sanguin dans le vaisseau sanguin ;
un dispositif de rétraction (120) est adapté pour déplacer la sonde de recueil de données (140) à travers le vaisseau sanguin à une vitesse sensiblement constante, la sonde de recueil de données (140) étant agencée pour recueillir des données tout en étant déplacée à la vitesse sensiblement constante ;
le dispositif de recueil de données (110) acquiert les données de vaisseau sanguin uniquement pendant l'intervalle alors que la sonde de recueil de données est déplacée à travers le vaisseau sanguin à la vitesse sensiblement constante en réponse à un déclenchement de sonde fourni une fois par battement cardiaque marquant un début de la partie cyclique se produisant entre des parties identifiables de chaque cycle cardiaque

2. Système selon la revendication 1, dans lequel les données de vaisseau sanguin comprennent des données de vaisseau sanguin appropriées pour identifier une forme d'un vaisseau sanguin, une densité du vaisseau sanguin, ou une composition du vaisseau sanguin.

3. Système selon les revendications 1 ou 2, dans lequel l'intervalle correspond à l'état d'un vaisseau sanguin formé de manière uniforme.

4. Système selon les revendications 1 ou 2, comprenant en outre un cathéter (342), la sonde de recueil de données étant fixée à une extrémité distale du cathéter.

5. Système selon la revendication 4, dans lequel la sonde de recueil de données comprend plusieurs transducteurs (344a, 344b, 344c, etc) espacés circonférentiellement autour de l'extrémité distale du cathéter et adaptés pour recevoir au moins les données de vaisseau sanguin.

6. Système selon la revendication 4, dans lequel la sonde de recueil de données comprend en outre au moins un transducteur adapté pour tourner et recevoir au moins les données de vaisseau sanguin.

7. Système selon les revendications 1, 2, ou 3, dans lequel le dispositif de surveillance cardiaque comprend un dispositif d'électrocardiographe (ECG).

8. Système selon les revendications 1, 2, ou 3, dans lequel le dispositif de recueil de données comprend un dispositif informatique programmable.

9. Système selon les revendications 1, 2, ou 3, dans lequel le dispositif de recueil de données comprend un dispositif intra-vasculaire à ultrasons (IVUS).

10. Système selon la revendication 8, dans lequel le dispositif de recueil de données comprend en outre un dispositif intra-vasculaire à ultrasons (IVUS).

11. Système selon la revendication 6, dans lequel le dispositif de recueil de données est en outre adapté pour commencer à acquérir les données de vaisseau sanguin, lorsque le au moins un transducteur est orienté en rotation dans une position prédéterminée.
